# EUROPEAN PATENT APPLICATION

(11) **EP 2 204 086 A1**
(43) Date of publication of application: **07.07.2010**
(21) Application number: 09150012.4
(22) Date of filing: 02.01.2009
(51) Int. Cl.: A01H 1/00, A01H 5/10, C12Q 1/68

(54) **Six row-type barley**

(71) Applicant: Leibniz-Institut für Pflanzengenetik und Kulturpflanzenforschung (IPK), 06466 Gatersleben (DE)
(72) Inventor: Gottwald, Sven, 06484 Quedlinburg (DE); Stein, Nils, 06484 Quedlinburg (DE)
(74) Representative: Maiwald Patentanwalts GmbH

(57) **Abstract**

The present invention relates to six row-type barley plants which comprise a nucleotide substitution on nucleotide position 856 within the nucleotide sequence coding for a Vrs protein and the use of these plants for the preparation of malt or beer. The present invention further relates to methods for obtaining such plants and methods for identifying such plants.

SEQUENCE LISTING

<110>  IPK (Leibniz-Institut für Pflanzengenetik und
       Kulturpflanzenforschung)

<120>  Six row-type barley

<130>  G 7528/RN

<160>  4

<170>  PatentIn version 3.3

<210>  1
<211>  2147
<212>  DNA
<213>  Hordeum vulgare

<400>  1
gtcataactc ggcaaacata gattagacag aattttctga gttcttatct agaggaactc      60

gatgaacttg aggcattgtc gaggttcttc ctttcaccga gtactttttt gcgtgtacta     120

ggcaaatata tgaagtttgt gagtttcgga tcaccaccga gtgcaagttt ggaccaaact     180

tgacaaatac ataagtttgg cgagctccga atgaaatgaa ctctgcaaaa gaatagaact     240

cggcgcaaaa ccagattcta atagtgtgtg aatttttggg ctgttttgta taaatatgat     300

gaaacttagt aaaatttcac tcaggtcaat gctaatgtgg agagtaaata aaaaatgaag     360

ggagtacttg gctgcatcat atgtttgccc ccgatcacct tcacatctcc ccgtccggac     420

ggcctggatc ggaaagcact cagccggagc cccgccggcg cttgccgttg ggtacctctg     480

ccacctattt atattacccc taggtctctc cctggagaca cgcactcccc tccttcaact     540

agtgctttgc ggcccgtggt cctcctctcg atccagttcc tgagcacacc aacaggcaac     600

agaacaacct accgtgtctc ccctccaatc tcctcacgat cccttctttc cctcagatcc     660

gaaccgaaag catggacaag catcagctct ttgattcatc caacgtggac acgactttct     720

tcgcggccaa tggtacacac gacgccgcgc gcgcccggtc tttgcgcatg cgatgatgca     780

gctgcagtag cttcagtttc accggccagg acacgcatgt gatgacgttt tttccattct     840

gtgtttgtat gtgcaggcac ggcgcagggg gataccagca agcagagggc gcggcgcagg     900

cggcggaggt cggcgaggtg cggcggaggg gatggtgacg gtggggagat ggacggagga     960

ggggacccca agaagcggcg gctcaccgac gagcaggccg agattctgga gctgagcttc    1020

cgggaggacc gcaagctgga gacagcccgc aaggtgtatc tggccgccga gctcgggctg    1080

gaccccaagc aggtcgccgt gtggttccag aaccgccgcg cgcgccacaa gaacaagacg    1140

ctcgaggagg agttcgcgag gctcaagcac gcccacgacg ccgccatcct ccacaaatgc    1200

cacctcgaga acgaggtatg cttgctcgca tacactcaca ctggcttaca tatggcgctg    1260

cacatctgca gttcctctcc gttcttgaac atgcttactg acaaacatat ggccagctgc    1320

tgaggctgaa ggagagactg ggagcgactg agcaggaggt gcggcgcctc aggtcggcag    1380

ctgggagcca cggggcatct gtggatggcg gacacgccgc tggcgccgtt ggcgtgtgcg    1440

gcgggagccc gagctcgtcc ttctcgacgg gaacctgcca gcagcagccg ggtttcagcg    1500

gggcagacgt gctggggcgg gacgatgacc tgatgatgtg cgtccccgag tggtttttag 1560

catgaattag agtttatgct ggctaagccg atagcagcgt ggtcgagtgt tttttagcat 1620

gaaatcagat ctccatctcc cataaaatag ccgagatagc tgctgccgcc gccaaatcct 1680

ctatagggct tcaagatcgg cagaaacctc tagaaatcat ctcccccctc cggaaaagtc 1740

gcctctattt gtctccattg cccgcgatgc agcatccggt atagctgcta agacaggccg 1800

cccctaaatc gtttctccag cgattttaat ctttggtttt tagcctgtat atatgggctg 1860

tgatttgaag ttgagacgag ctggacatca actgcacgct gatcgattac tattctagtt 1920

tggcatagtg ttaattaagt ttggatgatc tctaggcgtg cgttaagtat gtagatagtg 1980

ttgattaatg gcaaaagctt gcaagttaag tgtagtattg gcagctctct tgaagatcaa 2040

atatgatgtg tgttatcatt tgatgatata tattttactt cagccgtaaa tagtcttctt 2100

agggaagcac tgtccatgta tgtgctggta gttggcaatc atctttc 2147

<210> 2
<211> 2147
<212> DNA
<213> Artificial

<220>
<223> Vrs1 having a point mutation at position 856

<400> 2
gtcataactc ggcaaacata gattagacag aattttctga gttcttatct agaggaactc 60

gatgaacttg aggcattgtc gaggttcttc ctttcaccga gtactttttt gcgtgtacta 120

ggcaaatata tgaagtttgt gagtttcgga tcaccaccga gtgcaagttt ggaccaaact 180

tgacaaatac ataagtttgg cgagctccga atgaaatgaa ctctgcaaaa gaatagaact 240

cggcgcaaaa ccagattcta atagtgtgtg aatttttggg ctgttttgta taaatatgat 300

gaaacttagt aaaatttcac tcaggtcaat gctaatgtgg agagtaaata aaaaatgaag 360

ggagtacttg gctgcatcat atgtttgccc ccgatcacct tcacatctcc ccgtccggac 420

ggcctggatc ggaaagcact cagccggagc cccgccggcg cttgccgttg ggtacctctg 480

ccacctattt atattacccc taggtctctc cctggagaca cgcactcccc tccttcaact 540

agtgctttgc ggcccgtggt cctcctctcg atccagttcc tgagcacacc aacaggcaac 600

agaacaacct accgtgtctc ccctccaatc tcctcacgat cccttctttc cctcagatcc 660

gaaccgaaag catggacaag catcagctct ttgattcatc caacgtggac acgactttct 720

tcgcggccaa tggtacacac gacgccgcgc gcgcccggtc tttgcgcatg cgatgatgca 780

gctgcagtag cttcagtttc accggccagg acacgcatgt gatgacgttt tttccattct 840

gtgtttgtat gtgcaagcac ggcgcagggg gataccagca agcagagggc gcggcgcagg 900

cggcggaggt cggcgaggtg cggcggaggg gatggtgacg gtggggagat ggacggagga 960

ggggacccca agaagcggcg gctcaccgac gagcaggccg agattctgga gctgagcttc 1020

cgggaggacc gcaagctgga gacagcccgc aaggtgtatc tggccgccga gctcgggctg 1080

gaccccaagc aggtcgccgt gtggttccag aaccgccgcg cgcgccacaa gaacaagacg 1140

ctcgaggagg agttcgcgag gctcaagcac gcccacgacg ccgccatcct ccacaaatgc 1200

cacctcgaga acgaggtatg cttgctcgca tacactcaca ctggcttaca tatggcgctg 1260

cacatctgca gttcctctcc gttcttgaac atgcttactg acaaacatat ggccagctgc 1320

tgaggctgaa ggagagactg ggagcgactg agcaggaggt gcggcgcctc aggtcggcag 1380

ctgggagcca cggggcatct gtggatggcg gacacgccgc tggcgccgtt ggcgtgtgcg 1440

gcgggagccc gagctcgtcc ttctcgacgg gaacctgcca gcagcagccg ggtttcagcg 1500

gggcagacgt gctggggcgg gacgatgacc tgatgatgtg cgtccccgag tggtttttag 1560

catgaattag agtttatgct ggctaagccg atagcagcgt ggtcgagtgt tttttagcat 1620

gaaatcagat ctccatctcc cataaaatag ccgagatagc tgctgccgcc gccaaatcct 1680

ctatagggct tcaagatcgg cagaaacctc tagaaatcat ctcccccctc cggaaaagtc 1740

gcctctattt gtctccattg cccgcgatgc agcatccggt atagctgcta agacaggccg 1800

cccctaaatc gtttctccag cgattttaat ctttggtttt tagcctgtat atatgggctg 1860

tgatttgaag ttgagacgag ctggacatca actgcacgct gatcgattac tattctagtt 1920

tggcatagtg ttaattaagt ttggatgatc tctaggcgtg cgttaagtat gtagatagtg 1980

ttgattaatg gcaaaagctt gcaagttaag tgtagtattg gcagctctct tgaagatcaa 2040

atatgatgtg tgttatcatt tgatgatata tattttactt cagccgtaaa tagtcttctt 2100

agggaagcac tgtccatgta tgtgctggta gttggcaatc atctttc 2147

<210> 3
<211> 27
<212> DNA
<213> Artificial

<220>
<223> Primer "Vrs1-C-F"

<400> 3
tcaatgctaa tgtggagagt aaataaa 27

<210> 4
<211> 27
<212> DNA
<213> Artificial

<220>
<223> Primer "Vrs1-C-R"

<400> 4
atatgtttgt cagtaagcat gttcaag 27

## Description

The present invention relates to six row-type barley plants, which comprise a nucleotide substitution on nucleotide position 856 within the nucleotide sequence coding for a Vrs protein and the use of these plants for the preparation of malt or beer. The present invention further relates to methods for obtaining such plants and methods for identifying such plants.

Barley is an annual cereal grain, which serves as an animal feed crop and which is also used for malting and in health food. Barley is ranked fifth in quantity produced and in area of cultivation of cereal crops in the world, behind wheat, rye, corn and soy beans. In Germany it is ranked second behind wheat. The agriculturally most important parts of barley are the seeds (embryo and endosperm) which are used for the production of feed, food or malt.

In barley, two row-type and six row-type varieties can be distinguished based on the morphology of the spike. These varieties differ in yield, as the row type has a great impact on the number, shape and size of the grains.

The barley spike is composed of triplets (each with one central and two lateral spikelets) arranged alternately at rachis nodes. In six row-type barley cultivars all three spikelets are fully fertile and able to develop into grains, whereas in two row-type barley the lateral spikelets are reduced in size and are sterile. Furthermore, the reduced lateral spikelets in two row-type barley have greatly reduced stamens and a rudimentary ovary and stigma compared with those of the central spikelets. Two row-type barley has a lower protein content than six row-type barley and thus a higher content of fermentable sugars. Malting barley with a lower protein content shows more uniform germination, needs shorter steeping and has less protein in the extract that can make beer cloudy.

It has been discovered that the development of a six row-type spike is controlled by a single allele, *vrs1*, that is recessive to the dominant allele responsible for the two row-type spike (*Vrs1*) (Lundqvist et al. (1997) Barley Genet. Newsletter 26: 22-516; Lundqvist (1989) Hereditas 110: 27-233). It has further been shown that the presence of the recessive gene *vrs1* is by itself sufficient to cause two row-type barley to become six row-type barley. The *vrs1* gene was isolated and shown to encode a homeodomain-leucine zipper motif containing protein. Hence, the Vrs1 protein is a member of the HD-ZIP class of transcription factors (Komatsuda et al. (2007) Proc. Natl. Acad. Sci. USA 104(4): 1424-1429).

As the six row-type barley plants have a higher yield, it is desirable to produce six row-type barley plants. However, these plants should maintain the advantages of the two row-type plants, particularly the stableness and grain quality. At present, six row-type barley is mainly used in the USA and Canada, whereas it is not used in Germany and the rest of Europe. Hence, new alleles which are responsible for the six row-type in barley are also desirable for European breeders.

Therefore, there is still a need to develop six row-type barley plants which have an increased yield compared to two row-type barley plants, but which also maintain the advantageous features of two row-type barley plants.

Thus, it is an object of the present invention to provide six row-type barley plants which maintain the advantageous features of two row-type barley plants.

Another object of the present invention is to provide a method for obtaining six row-type barley plants and methods to identify six row-type barley plants.

These and other objects of the invention are attained by the subject-matter of the independent claims. Advantages and embodiments are defined in the independent claims.

### Summary of the invention

The present inventors have surprisingly found in a TILLING (Targeting Induced Local Lesions IN Genomes) approach as described in the examples of the present application that a single point mutation on position 856 of the nucleic acid sequence coding for a Vrs protein is sufficient to obtain six row-type barley plants. This mutation is within the 5' splicing site of the second exon and leads to a silencing of *Vrs1* gene function. Hence, this finding may be used to produce plants having this point mutation in a non-transgenic approach.

Hence, the present invention provides barley plants comprising a nucleotide substitution on nucleotide position 856 of the nucleic acid sequence coding for a Vrs protein, the nucleotide numbering referring to the nucleic acid sequence depicted in SEQ ID NO. 1.

Preferably, the nucleotide substitution is a G to A substitution. Even more preferably, the nucleic acid sequence comprising the nucleotide substitution on nucleotide position 856 is the nucleotide sequence depicted in SEQ ID NO. 2.

Further subjects of the present invention are hybrids, progeny and parts of the barley plant comprising a nucleotide substitution on nucleotide position 856 of the nucleic acid sequence coding for a Vrs protein, the nucleotide numbering referring to the nucleic acid sequence depicted in SEQ ID NO. 1.

In a further aspect, the present invention relates to the use of the barley plant of the present invention, the hybrids, the progeny or the parts of said plants for the manufacture of a malt or a beverage. Preferably, the beverage is beer.

In still a further aspect, the invention provides a method for obtaining six row-type barley plants comprising the steps of
a) mutagenizing barley seed;
b) regenerating barley plants from the seeds; and
c) identifying barley plants comprising a nucleotide substitution on nucleotide position 856 of the nucleic acid sequence coding for a Vrs protein, the nucleotide numbering referring to the nucleic acid sequence depicted in SEQ ID No. 1.

Preferably, the barley plants according to the present invention are identified by amplifying a nucleic acid sequence comprising nucleotide 856 of the nucleotide sequence coding for a Vrs protein from a nucleic acid sample obtained from a barley plant and determining whether the nucleic acid sequence coding for a Vrs protein comprises a nucleotide substitution on position 856, the nucleotide numbering referring to the nucleic acid sequence depicted in SEQ ID No.1.

In still a further aspect, the present invention provides a method for identifying six row-type barley plants comprising identifying plants which comprise a nucleotide substitution on nucleotide position 856 of the nucleic acid sequence coding for a Vrs protein, the nucleotide numbering referring to the nucleic acid sequence depicted in SEQ ID NO. 1.

Further, the present invention relates to a primer set for amplifying a nucleic acid sequence comprising nucleotide 856 of the nucleic acid sequence coding for a Vrs protein, the nucleotide numbering referring to the nucleic acid sequence depicted in SEQ ID NO. 1, said primer set comprising a nucleic acid molecule comprising the nucleic acid sequence according to SEQ ID NO. 3 and a nucleic acid molecule comprising the nucleic acid sequence according to SEQ ID NO. 4.

Finally, the present invention relates to an isolated nucleic acid molecule comprising a nucleic acid sequence selected from the group consisting of:
a) a nucleic acid sequence according to SEQ ID No. 2;
b) nucleic acid sequences hybridising to a complementary strand of the nucleic acid sequence according to SEQ ID No. 1 under stringent conditions and having a nucleotide substitution on position 856 of the nucleic acid sequence coding for a Vrs protein, the nucleotide numbering referring to the sequence depicted in SEQ ID No. 1; and
c) nucleic acid sequences which are at least 70 % homologous to the nucleic acid sequence shown in SEQ ID No. 1 and which have a nucleotide substitution at position 856 of the nucleic acid sequence coding for a Vrs protein, the nucleotide numbering referring to the sequence depicted in SEQ ID No. 1.

### Brief description of the drawings

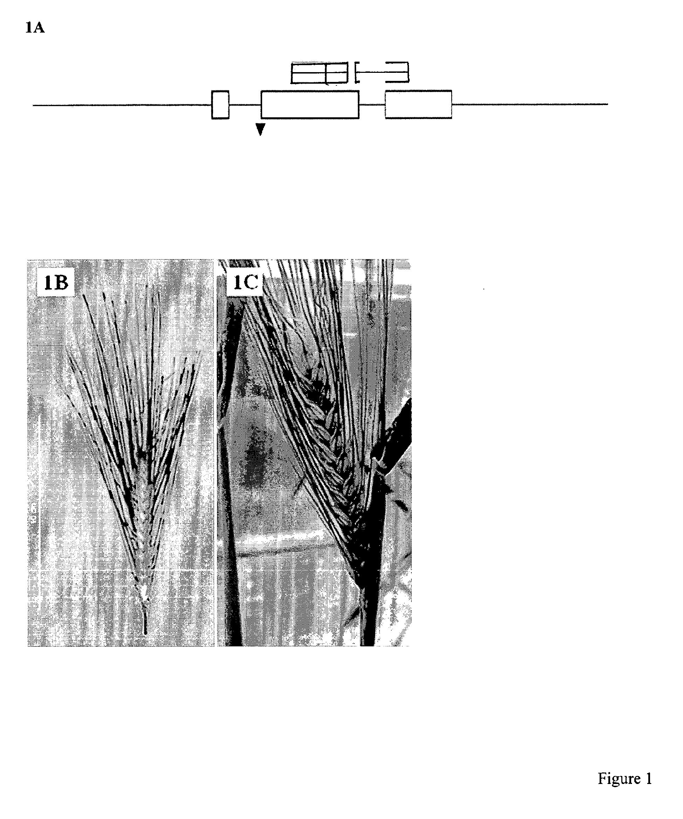

- Figure 1:: A) Vrs1 gene model showing the position of the nucleotide substitution on position 856 in the 5' splicing site of exon II (arrow). The lower rectangles show the gene structure comprising exons I to III, the upper rectangles show the functionally relevant HD-ZIP domain.
B) Grain phenotype of a two row-type barley wild type plant.
C) Grain phenotype of the six row-type barley plant of the present invention.

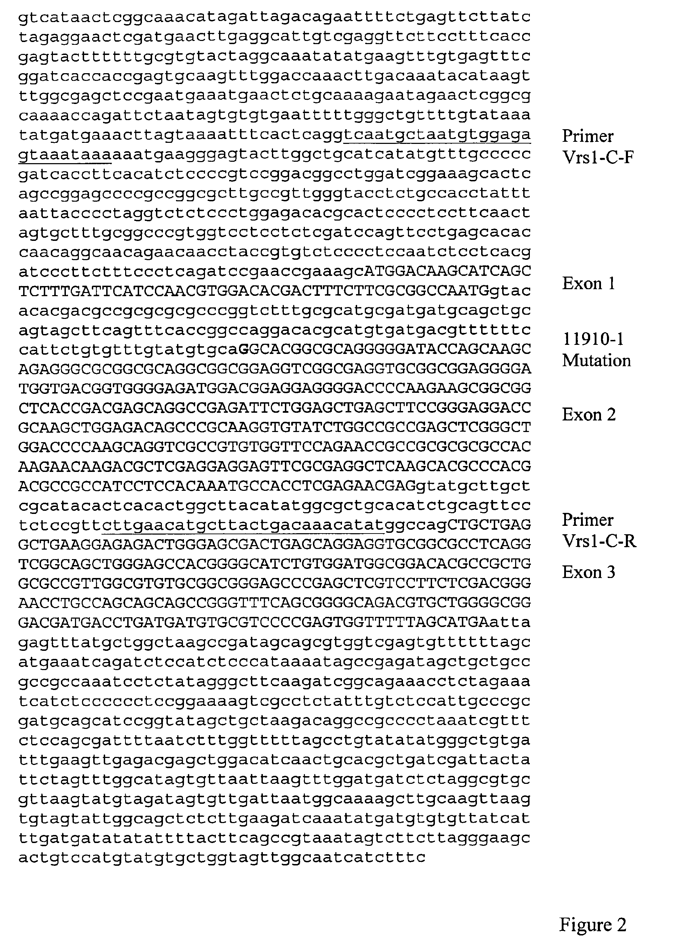

- Figure 2:: Nucleic acid sequence comparison showing the G to A nucleotide substitution on position 531 of a 985 bp *Vrs1*-C fragment obtained by amplifying the Vrs1 nucleotide sequence with primers having the nucleic acid sequences according to SEQ ID Nos. 3 and 4. Position 531 corresponds to position 856 within the full-length nucleotide sequence coding for a Vrs protein according to SEQ ID No. 1.

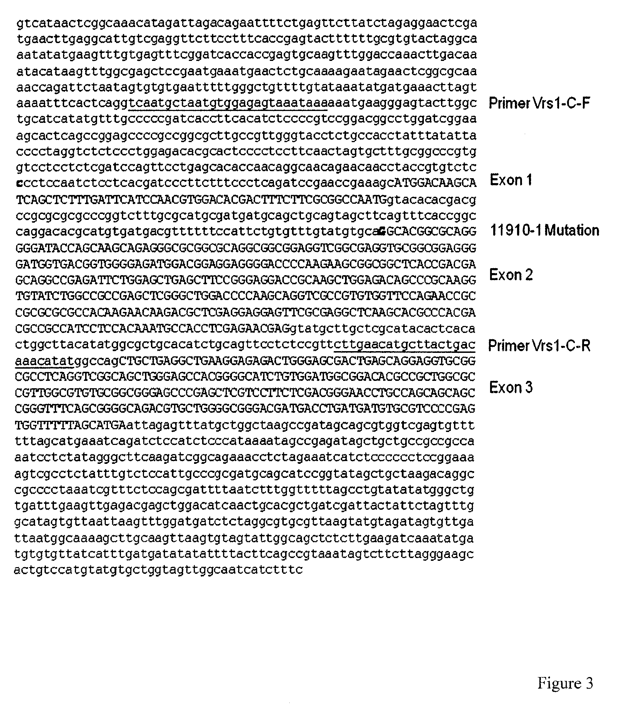

- Figure 3:: Sequence and structure of the gene coding for the Vrs1 protein. The nucleotide sequence of the exons is depicted in upper case letters, whereas the nucleotide sequence of the introns is shown in lower case letters. The nucleotide substitution on position 856 of the nucleotide sequence is shown as bold letter. The sequences to which the primers according to SEQ ID Nos. 3 and 4 anneal are underlined.

### Detailed description of the invention

The term "barley plant" within the meaning of the present invention is intended to comprise plants of the genus *Hordeum*, preferably of the species *Hordeum vulgare.* The plants may belong to cultivar barley, i. e. *Hordeum vulgare ssp. vulgare* or to wild barley, i.e. *Hordeum vulgare ssp. spontaneum*. Hybrid or crossbred varieties between cultivar and wild barley are also acceptable.

Preferably, the barley plants are elite plants. An "elite plant" within the meaning of the present invention is a plant which is sufficiently homogenous to be used for commercial grain production, but which might also be used for further breeding steps.

Examples for elite plants of the species *Hordeum vulgare* are the European malting cultivar "Barke", "JB Maltasia", "JB Flavour", "Scarlett", "Mamie", "Felicitas", "Odessa", "Denise" and "Isotta".

The "six row-type" or "six-rowed" phenotype is intended to mean a phenotype in which the degree of lateral spikelet development within a node on the rachis is comparable to that of the central spikelet. In contrast, the "two row-type" or "two-rowed" phenotype refers to a phenotype in which lateral spikelets do not develop within a node on the rachis. The degree of lateral spikelet development is comparable to that of the central spikelet if the lateral spikelets are fertile and/or if the size of the lateral spikelets is at least 50% or 60%, preferably at least 70% or 80%, more preferably at least 90% or 95% and most preferably at least 98%, 99% or 100% of the size of the central spikelet.

The "rachis" is the main axis of the inflorescence of cereals to which the spikelets are attached. The rachis is composed of nodes and internodes, wherein the spikelets are attached to the nodes.

"Spikelets" are small inflorescences bearing one or more florets along with a set of miniature bractlike leaves. The fertile spikelets can later develop into kernels.

Further details on the anatomy of the barley spike are known to the expert and can be obtained from textbooks such as Barley and Malt: Biology, Biochemistry and Technology, A.H. Cook, Ed., Academic Press, New York, 1962.

The term "nucleotide substitution" means that a nucleotide on a specific position within a given nucleic acid sequence is replaced with another nucleotide, for example a G residue on a specific position may be replaced with A, T or C. The nucleotide substitution which leads to the six row-type phenotype of the present invention is located on position 856 of the nucleic acid sequence coding for a Vrs protein, the nucleotide numbering referring to the nucleic acid sequence depicted in SEQ ID NO. 1. This position represents the 5' splicing site of the second exon. Hence, the point mutation leads to the expression of a non-functional Vrs protein and to the development of the six row-type phenotype. Preferably, the nucleotide substitution on position 856 of the nucleic acid sequence coding for a Vrs protein is a G to A substitution.

The term "nucleic acid sequence coding for a Vrs protein" within the meaning of the present invention is not intended to mean that the sequence comprising the nucleotide substitution actually has to be transcribed and translated into the Vrs protein, but it is only intended to designate the reference sequence to locate the nucleotide substitution of the present invention. The reference nucleic acid sequence for the numbering of the nucleotides, in particular nucleotide 856, which is substituted in the barley plants of the present invention, is the wild-type Vrs coding sequence which is shown in SEQ ID NO. 1 and which comprises a G on position 856 of SEQ ID No.1.

The present invention is not intended to be limited to nucleic acid sequences comprising only the nucleotide substitution on position 856, but the nucleotide sequences may also comprise further modifications with respect to the nucleotide sequence according to SEQ ID NO. 1 such as further nucleotide substitutions, deletions or additions, as long as the nucleotide substitution on nucleotide position 856 in the nucleic acid sequence coding for a Vrs protein is present.

In a preferred embodiment, the seeds of the barley plant according to the invention are deposited at the "national Collections of Industrial Food and Marine Bacteria" (NCIMB, Aberdeen, UK) under the Accession number 41602 (date of original deposit: 16 December 2008). The designation of the deposited line is M4 line 11910-1.

The invention further relates to hybrid plants of the barley plants of the present invention. Hybrid plants are the progeny of two genetically non-identical parents which are produced by cross-pollination of genetically different parental lines. The hybrid progeny shows the so-called "heterosis effect", which means that they display superior plant growth, feed yield and a pronounced stress tolerance in comparison to both parental lines. For naturally self-pollinating plants, the plant must be male sterilized during the crossing process in order to avoid self-fertilization and to facilitate hybrid breeding. Male sterility may be achieved by mechanical, genetic or chemical means. The hybrid plants of the present invention also comprise a nucleotide substitution on nucleotide position 856 in the nucleic acid sequence coding for a Vrs protein, the nucleotide numbering referring to the sequence depicted in SEQ ID No. 1.

The present invention further relates to progeny of the barley plants of the present invention, which progeny may be obtained by both generative and vegetative production. The progeny within the meaning of the present invention also comprises a nucleotide substitution on nucleotide position 856 in the nucleic acid sequence coding for a Vrs protein, the nucleotide numbering referring to the nucleic acid sequence depicted in SEQ ID No. 1.

The present invention further relates to parts of the barley plants of the present invention, for example, stems, leaves, roots, flowers, seeds, grains, fruits or buds. Preferably the part of the barley plant is the grain. The parts of the barley plant of the present invention also comprise a nucleotide substitution on nucleotide position 856 in the nucleic acid sequence coding for a Vrs protein, the nucleotide numbering referring to the nucleic acid sequence depicted in SEQ ID No. 1.

The barley plant of the present invention, its hybrids, progeny or parts thereof may be used in various ways, e.g. for the manufacture of a malt or a beverage, as ornamental plant, for animal feed and for the manufacture of food such as soups and stews.

The beverage manufactured using the barley plant of the present invention may be beer, whiskey, barley water, mogicha and coffee substitutes.

Preferably, the barley plant of the present invention is used for the manufacture of a malt or a beer.

The "malt" is intended to mean a barley grain which was germinated for a limited period of time and then dried.

The term "beer" is intended to comprise all kinds of beer which involve the use of barley malt, such as ales, lagers and weissbier.

The preparation of a malt from barley grain typically comprises at least the steps of steeping, germination and kiln-drying.

"Steeping" involves the addition of water and oxygen to the barley grain to create growth conditions for germination.

"Germination" involves the secretion of enzymes from the grain to degrade the protein, starch and cell walls of the endosperm.

"Kiln-drying" is the interruption of germination, moisture extraction and formation of aroma.

The malt may then be used to make beer. The preparation of beer from the malt typically comprises the following steps:
a) crushing of malted barley into coarse flour (the so-called grist);
b) addition of warm water to form mash by breaking down the starch into sugars;
c) lautering (separation of wort from spent solids);
d) boiling the wort with hops;
e) clarifying, cooling and aerating the wort;
f) fermenting wort with yeast to alcohol and carbon dioxide; and
g) maturing and clarifying the beer.

The present invention further relates to a method for obtaining six row-type barley plants which involves the step of mutagenising barley seed.

The term "mutagenising" is intended to mean that one or more mutations are introduced into the genome of an organism such as barley. The mutations may comprise nucleotide substitutions, additions or deletions.

Suitable mutagens for mutagenising the barley seeds are, for example, chemical mutagens such as sodium azide, iso-propylmethanesulfonate, ethyl methanesulfonate, glycidol, ethylene imine, ethyl hydroxyethanesulfonate, methyl methanesulfonate, butyl methanesulfonat, propanedisulfonic acid diethyl ester and N-ethyl-N-nitrosourea. The seeds may also be mutagenised by radiation with neutrons, X-rays or gamma-rays.

Preferably, the seeds are mutagenised by the treatment with ethyl methanesulfonate (EMS).

Furthermore, the nucleotide substitution on position 856 of the *vrs1* gene may also be introduced by site-directed mutagenesis, for example as described in EP 0 733 059.

Optionally, barley plants are regenerated from the mutagenized seeds. Usually, the regeneration from the seeds involves the germination of the seeds.

The steps of identifying barley seeds comprising a nucleotide substitution on nucleotide position 856 of the nucleic acid sequence coding for a Vrs protein preferably involves the steps of amplifying a nucleic acid sequence comprising nucleotide 856 of the nucleic acid sequence coding for a Vrs protein from a nucleic acid sample obtained from a barley plant and determining the nucleic acid sequence of the amplified sequence.

The nucleic acid samples may be obtained from any suitable tissue, such as leaves, grains or roots by any process which is known in art. For example, the tissue is cut into small pieces, nitrogen is added to these pieces and the tissue is ground thoroughly, before it is transferred to a tube. Then, DNA extraction buffer is added to the ground tissue and the sample is incubated with the buffer for a certain time. Afterwards the nucleic acids are extracted with alcohol and precipitated.

The term "amplifying" means that a certain nucleic acid sequence present in the nucleic acid sample is copied several times, preferably by use of suitable primers which hybridise on the 5' and 3' end of the nucleic acid sequence to be amplified, e.g. a nucleic acid sequence comprising the nucleotide on position 856 of the nucleic acid sequence coding for a Vrs protein. Amplification with these primers, e.g. the primers having the SEQ ID Nos. 3 and 4, will yield a nucleic acid molecule comprising the nucleotide on position 856 of the nucleic acid sequence coding for a Vrs protein.

The amplification process is preferably a PCR process which comprises several cycles of denaturing the nucleic acids within a sample, annealing of the primers, and elongation of the nucleic acid molecule.

Subsequently, it is determined whether the nucleic acid sequence coding for a Vrs protein comprises a nucleotide substitution on position 856, the nucleotide numbering referring to the sequence depicted in SEQ ID No.1.

For example, the nucleic acid sequence of the amplified DNA sequence may be determined by any method known in the art of DNA sequencing, e.g. by the chain termination developed by Sanger which involves the use of labeled dideoxy nucleotide triphosphates as chain terminators.

A person skilled in the art knows other methods for detecting a point mutation within a nucleic acid sequence, such as hybridization with radioactively labeled allele-specific oligonucleotides (ASO), restriction fragment length polymorphism (RFLP), allele-specific amplification (ASA), allele-specific hybridization, amplified fragment-length polymorphism (AFLP), single nucleotide primer extension and oligonucleotide ligation assay (OLA). These techniques are known to the expert and reviewed for example in Nollau and Wagener (1997) Clinical Chemistry 43(7): 1114-1128; Drabek (2001) Electrophoresis 22(6):1024-1045 and Kim and Misra (2007) Annu. Rev. Biomed. Eng. 9: 289-320. Furthermore, kits for SNP detection have been developed by different companies, such as the AcycloPrime^{™} II-FP SNP Detection Kit of Perkin Elmer or the Readit^{™} system of Promega.

Because of the phenotype of the six row-type plants which differs considerably from the phenotype of the two row-type plants (see Figures 1B and C), it is also possible to distinguish six row-type plants from two row-type plants by visual inspection. Optionally, this visual inspection may be followed by one of the molecular biological methods listed above.

Although a non-transgenic approach for producing the plants of the present invention is preferred, it is also possible to transform a barley plant with a recombinant nucleic acid molecule comprising a nucleic acid sequence with a nucleotide substitution at position 856 of the nucleic acid sequence coding for a Vrs protein, the nucleotide numbering referring to the sequence depicted in SEQ ID No. 1. The steps of preparing the recombinant nucleic acid molecule and transforming it into plant cells are well-known to the expert and guidance may be obtained from standard textbooks such as Sambrook and Russell, Molecular Cloning - A laboratory manual, third edition 2001, Cold Spring Harbor Laboratory Press, Cold Spring Harbor, New York. In this approach, it might be necessary to knock out the endogenous *vrs1* gene by methods known to the expert, for example RNA interference or post-transcriptional silencing.

The present invention further relates to an isolated nucleic acid molecule comprising a nucleic acid sequence selected from the group consisting of:
a) a nucleic acid sequence according to SEQ ID No. 2;
b) nucleic acid sequences hybridising to a complementary strand of the nucleic acid sequence according to SEQ ID No. 1 under stringent conditions and having a nucleotide substitution at position 856 of the nucleic acid sequence coding for a Vrs protein, the nucleotide numbering referring to the sequence depicted in SEQ ID No. 1; and
c) nucleic acid sequences which are at least 70 % homologous to the nucleic acid sequence shown in SEQ ID No. 1 and which have a nucleotide substitution at position 856 of the nucleic acid sequence coding for a Vrs protein, the nucleotide numbering referring to the sequence depicted in SEQ ID No. 1.

According to the present invention, the term "homologous" is generally understood to denote that the nucleic acid sequence of a DNA molecule is identical to the Vrs1 nucleic acid sequence by at least 70%, preferably by at least 80% or 85%, especially preferably by at least 90%, 92% or 94%, particularly preferably by at least 95%, 96% or 97% and most preferably by at least 98% or 99%. Preferably, homology is determined over the entire sequence length of the Vrs1 nucleic acid sequence.

Nucleic acid molecules are identical if they have identical nucleotides in the same 5' to 3' order.

Thus, homology is preferably calculated over the entire nucleic acid sequence region. The person skilled in the art knows programs based on different algorithms for comparing different sequences. Herein, the algorithms by Needleman and Wunsch, or Smith and Waterman yield particularly reliable results. For said sequence comparisons, for example, the program PileUp (Feng and Doolittle, J. Mol. Evolution. (1987) 25: 351-360; Higgins et al. (1989) CABIOS 5: 151-153) or the programs Gap and Best Fit (Needleman and Wunsch (1970) J. Mol. Biol. 48: 443-453 and Smith and Waterman (1981) Adv. Appl. Math. 2: 482-489), which are contained in the GCG Software Package by the Genetics Computer Group (575 Science Drive, Madison, Wisconsin, USA 53711), can also be used.

A further object of the present invention are nucleic acid molecules, which hybridize under stringent conditions with, or are substantially complementary to, those nucleic acid molecules coding for a Vrs1 protein. The term "complementarity" describes the capability of a nucleic acid molecule of hybridizing with another nucleic acid molecule due to hydrogen bonds formed between complementary bases. The person skilled in the art is aware of the fact that two nucleic acid molecules do not have to have a 100% complementarity in order to be able to hybridize with each other. Preferably, a nucleic acid sequence, which is supposed to hybridize with another nucleic acid sequence, is complementary to the latter by at least 40%, by at least 50%, by at least 60%, preferably by at least 70%, especially preferably by at least 80%, also especially preferably by at least 90%, particularly preferably by at least 95%, and most preferably by at least 98% or 100%.

Stringent *in vitro* hybridization conditions are known to the person skilled in the art and can be taken from the literature (see, for example, Sambrook and Russell, *vide supra*).

The term "specific hybridization" relates to the fact that a molecule preferably binds to a specific nucleic acid sequence under stringent conditions, provided that said nucleic acid sequence is part of a complex mixture of, for example, DNA or RNA molecules.

Thus, the term "stringent conditions" relates to conditions, under which a nucleic acid sequence preferably binds to a target sequence, but not, or at least in a significantly reduced manner, to other sequences.

Stringent conditions are dependent on the circumstances. Longer sequences hybridize specifically at higher temperatures. In general, stringent conditions are selected in such a way that the hybridization temperature is about 5°C below the melting point (Tₘ) for the specific sequence at a defined ionic strength and a defined pH value. Tₘ is the temperature (at a defined pH value, a defined ionic strength, and a defined nucleic acid concentration), at which 50% of the molecules, which are complementary to a target sequence, hybridize with said target sequence. Typically, stringent conditions comprise salt concentrations between 0.01 and 1.0 M sodium ions (or ions of another salt) and a pH value between 7.0 and 8.3. The temperature is at least 30°C for short molecules (for example, for those comprising between 10 and 50 nucleotides). In addition, stringent conditions may comprise the addition of destabilizing agents, like for example formamide. Typical hybridization and washing buffers are of the following composition.

| *Pre-hybridization solution:* | |
|---|---|
| | 0.5 % SDS |
| | 5 x SSC |
| | 50 mM NaPO₄, pH 6.8 |
| | 0.1% Na pyrophosphate |
| | 5 x Denhardt's Reagent |
| | 100 µg/ml salmon sperm |
| | |
| *Hybridization solution:* | Pre-hybridization solution |
| | 1 x 10⁶ cpm/ml probe (5-10 min, 95°C) |
| | |
| *20 x SSC:* | 3 M NaCl |
| | 0.3 M sodium citrate |
| | ad pH 7 with HCl |
| | |
| *50 x Denhardt's Reagent:* | 5 g Ficoll |
| | 5 g polyvinyl pyrrolidone |
| | 5 g Bovine Serum Albumin |
| | ad 500 ml A. dest. |

A typical hybridization procedure is conducted as follows:

| | |
|---|---|
| *Optional:* | washing the blot 30 min in 1 x SSC / 0.1% SDS at 65°C |
| *Pre-hybridization:* | at least 2 h at 50-55°C |
| *Hybridization:* | overnight at 55-60°C |

| | | | |
|---|---|---|---|
| *Washing:* | 5 min | 2 x SSC / 0.1% SDS | Hybridization temp. |
| | 30 min | 2 x SSC / 0.1% SDS | Hybridization temp. |
| | 30 min | 1 x SSC / 0.1% SDS | Hybridization temp. |
| | 45 min | 0.2 x SSC / 0.1% SDS | 65°C |
| | 5 min | 0.1 x SSC | Room temp. |

In a preferred embodiment, the nucleotide substitution in the isolated nucleic acid molecule is a G to A substitution.

The present invention is further illustrated by the following examples which are not intended to limit the scope of the invention in any way.

### Examples

### 1) Seed material

Seeds of *Hordeum vulgare* L. cultivar 'Barke' (obtained from: Saatzucht Josef Breun GdbR, Herzogenaurach, Germany) were used for chemical mutagenesis. 'Barke' is a spring-type, two row-type, European malting variety. 'Barke' was generated by cross-breeding between the two row-type spring barley varieties 'Alexis' (BSA 1102) x 'Libelle' (1256) in 1996. The pedigrees of both 'Barke' parents are: 'Alexis' ('Br1747' x 'Rupee') x 'Br1622') from 1990, and 'Libelle' ('Br 1622' x 'Trumpf') from 1968.

### 2) Chemical mutagenesis

Barley seeds were treated with different concentrations of the chemical mutagen ethylmethane sulfonate (EMS). Batches of ∼1,600 seeds each were filled into 2000 ml glass flasks and pre-soaked in 500ml de-ionised water (ddH2O) for 4h at room temperature (20-25°C). Then water was replaced by 350 ml of EMS solution [20 - 60 mM in ddH2O] and seeds were gently shaked (125rpm, a tabletop shaker) for 16h at room temperature. Subsequently, the EMS solution was collected for decontamination and the seeds were washed two times with 250 ml of 200mM sodium thiosulfate (30min each step) and subsequently two times with 11 of ddH20 (first for 30min, second for 1h). After removing the supernatant seeds were transferred to trays covered with Whatman paper and air-dried for 16h at 4°C prior to sowing.

### 3) Creation of the TILLING population

### a) Cultivation of M1

M1 plants obtained by mutagenesis were greenhouse-grown till maturity in 299-well potting plates placed on flooding tables. 23 and 29 days after sowing seedlings were sprayed with fertilizer solution (0.2% Wuxal, sodium-phosphate-potassium 8-8-6, AGLUKON GmbH & Co.KG, Düsseldorf, Germany). Subsequently, plants were watered via flooding the tables every second or third day in summer and winter times, respectively, with water-soluble Hakaphos blue (sodium-phosphate-potassium 15-10-15 & nutrient salts, COMPO GmbH & Co.KG, Münster, Germany). Plants were allowed to self-pollinate and the main spike of each plant was harvested as a source of M2 seeds. Every M1 generation was evaluated regarding germination and sterility rates. Germination was measured as percentage of developed seedlings, and sterility was measured as seed set per harvested main spike. Germination data were collected 8 days after sowing for EMS treatments between 20 and 40 mM and 12 days after sowing for concentrations between 45 and 60mM due to the delayed development of those seedlings.

### b) Cultivation of M2

From each M1, a progeny of maximum ten M2 individuals was cultivated to tillering stage (BBCH 30, Meier and Bleiholder: BBCH-Skala : phänologische Entwicklungsstadien wichtiger landwirtschaftlicher Kulturen, einschließlich Blattgemüse und Unkräuter, Agrimedia 2006). Subsequently, plants were evaluated and one or two individuals of each family were selected based on viability to ensure a sufficient seed set for further cultivation. M2 plants were cultivated as batches either under greenhouse (1,000 M2 families) or field (3,780 M2 families) conditions. M2 seedlings were regularly monitored for the presence of chlorophyll defects and other visible mutant phenotypes. For the analysis of chlorophyll mutant frequencies as a measuring method the relative frequencies as mutations per germinated 100 M2 plants were calculated according to Gaul (1960) Genetica Agraria 12: 297-318. All phenotypes were scored in reference to parent cultivar 'Barke'. M3 seed from individual M2 mutants were collected, catalogued, vacuum-packed and stored at 4°C until used as a resource for phenotyping.

### c) Cultivation of M3:

M3 progeny of identified mutants was grown for genotyping and for the determination of visible mutant phenotypes. Further evaluation of M3, in the purpose of "forward genetics" mutant identification, was performed on a subset of M3 families (16 seeds per single M2 individual) derived from concentrations between 20 and 40 mM EMS. These were cultivated under field conditions. Plants were scored for visible phenotypes during seedling stage, tillering, awn emergence, time of heading and final maturity. All phenotypes were scored in reference to parent cultivar 'Barke'.

### 4. Genomic DNA isolation

Genomic DNA was prepared from young leaves after lyophilization of tissue. Twenty to thirty milligram of lyophilized leaf tissue were milled in 2 ml tubes and DNA was extracted using a procedure essentially according to Doyle and Doyle (Doyle and Doyle (1987) Phytochem. Bull. 19: 11-15; Doyle and Doyle (1990) Focus 12/1: 13-15). The DNA was subsequently transferred to 96-well plates and concentrations were measured with a FLX 800 Microplate Fluorescence Reader (384 square well plates; BIO-TEC Instruments, Inc.) using Hoechst 33258 dye, blue (Sigma, Deisenhofen, Germany) according to manufacturer's instructions (Version 04/2004). An aliquot of DNA was diluted for PCR-purpose to a final concentration of 20 ng/µl and diluted DNAs were arranged in 8-fold 2D-pools for mutation screening.

### 5. Cel I-based mutation screening

### a) Primers, PCR

Oligonucleotides for PCR were designed either using the Primer3 software (http://frodo.wi.mit.edu/cgi-bin/primer3/primer3_www.cgi) directly or via the program CODDLE (Codons Optimized to Discover Deleterious Lesions; http://www.proweb .org/coddle/). Gene-specific primer design was either based on genomic or EST sequence information for the candidate genes. The primer sequences are shown in SEQ ID Nos. 3 and 4. Amplification of DNA with these primers yields a 985 bp fragment wherein the G to A substitution of the present invention is located on position 531 of the nucleotide sequence. Unlabeled and identical primers labeled at the 5'end with the fluorescent dye IRD700 (forward) or IRD800 (reverse), respectively, were mixed and used in PCR amplification as follows: 3:2 (labeled : unlabeled) ratio for the 100µM IRD700-labeled forward primer and 3:1 (labeled : unlabeled) ratio for the 100 µM IRD800-labeled reverse primer. Primers were designed to fit melting temperatures between 60°C and 70°C.

PCR amplification was carried out in a 30 µl volume containing 20 ng of individual pooled DNA, 3 µl of 10x buffer (Qiagen, Hilden, Germany), 1.2 µl of 5mM dNTPs, 0.9µl primers (10 µM/µl), 0.8 U Taq polymerase (Qiagen, Hilden, Germany). PCR was conducted using a thermal cycler (Applied Biosystems 9800 Fast Thermal Cycler, Foster City, USA) as follows: heat denaturation at 95°C for 3min, followed by 8 cycles of touchdown PCR (94°C for 30s, annealing at primer Tm +3°C to Tm +4°C for 30s decrementing -1°C per cycle and extension at 72°C for 1 to 1.30min [for 300 to 1.500bp products]); 35 cycles of: heat denaturation at 94°C for 30s, annealing at primer Tm -4°C to Tm -5°C for 30s decrementing -1°C per cycle and extension at 72°C for 1 to 1.30min; 72°C for 10min. The amplification step was followed by the heteroduplex formation: inactivation and denaturation at 99°C for 10min; and a re-annealing process of 70 cycles for 20s at 70°C to 49°C, decrementing 0.3°C per cycling.

### b) Cell nuclease mismatch cleavage assay

After PCR amplification, samples were incubated for 45min at 45°C in a 10 µl volume of 50-100 ng DNA with 0.06 U of Surveyor *Cell* enzyme (Transgenomics, Omaha, USA) in a 10x buffer (10mM HEPES (pH 7.0), 10 mM KCl, 10 mM MgCl4*7H20, 0,002% Triton X-100 and 10 µg/ml BSA). *Cell* Digestion was stopped by adding 5µl of 75mM EDTA (pH 8.0) followed by freezing (-20°C) of the probes for 30min.

Precipitation of 30 µl samples in 96-well PCR plates was carried out by adding 3 M Na-Acetate (pH 5.2) and 75 µl EtOH (99.8%), subsequent shaking for 15 - 20min, and centrifugation for 30min at 4470,6 x g. The supernatant was discarded by turning the plates on top of a stack of filter paper and plates were centrifuged face-down on filter paper for 1min at 497 x g. Precipitated samples were washed in 100µl ethanol (75%) at room temperature (20 - 25°C) while being centrifuged for 30min at 4470,6 x g. Removal of the supernatant was performed as described above. Finally, samples were dried for 20min at room temperature, resuspended in 8 µl formamide loading buffer (33% (w/v) deionised formamide (Roth, Karlsruhe, Germany), 25 mM Tris (pH 7.5), 25 mM EDTA and ∼0.02% (w/v) bromphenol blue) with constant shaking (300 rpm) for 5-10min, denaturated for 5min at 95°C and subsequently placed on ice until electrophoresis. Electrophoresis of the denatured DNA samples was performed (6.0% Long Ranger polyacrylamide slab gels, FMC Corporation, composition according to manufacturer's data; 1x TBE buffer: 89 mM Tris Base, 89 mM Boric Acid, 2 mM Na2EDTA*2H20) at 1,500V, 40 mA and 40 W settings on a Li-COR 4300 DNA Analyser (LI-COR Biosciences, Lincoln, NE). Gel images were visualised using the GelBuddy software (http://www.proweb.org/gelbuddy; Zerr and Henikoff 2005) and eye-inspected for presence of cleavage products.

TILLING screens were conducted by using a *Cell*-based hetero-duplex analysis for mutation detection. The *Cell*-based assay relies on using the LI-COR two laser / PA-slabgel system. The optimal mutation detection procedure and pool size for 'Barke' DNA was determined by performing test screenings using known mutations in the *Hv-eIF4E* gene (Stein et al. (2005) Plant J. 42: 912-922) of the barley genotypes 'IGRI' and 'Franka'. Thereby, it was found that for pooled DNA a 1:10 dilution of *Cell* in a specific buffer solution combined with a 45min digest at 45°C provides an optimal ratio between background and signal strength. The 8-fold arrangement was found to be most reliable, although in the tested 16-fold pools mutations were still detectable. To improve the mutation screening, the population was screened in a two-dimensional format. In each 96-well pool plate the DNA of 384 M2 individuals were arranged in row and column pools. As each sample is present in two different pools, any mutation is optimally detected twice.

### c) DNA sequencing and sequence data processing

For confirmation of putatively mutated loci, amplicons of the respective target gene were generated from putative mutants by utilising the same PCR conditions as established for CEL I analysis and exclusively unlabeled primers. Amplicons were purified by ultrafiltration using a QIAvac 96 vacuum-driven (Qiagen, Hilden, Germany) processed with a NucleoFast-96 PCR Plate (MACHEREY-NAGEL GmbH & Co. KG, Düren, Germany) and directly cycle-sequenced using ABI Big Dye terminator v3.1 sequencing standard kit according to manufacturers protocol (Applied Biosystems, Foster City, USA). Sequencing reactions were resolved on a 96-capillary sequencing device (3730xl DNA Analyzer, Applied Biosystems, Foster City, USA). Sequences were aligned against the "wild-type" reference of cultivar Barke by utilising Sequencher 4.6 software (Gene Codes, Ann Arbor, MI).

Genes were routinely analysed using the program CODDLE (Codons Optimized to Discover Deleterious Lesions; http://www.proweb.org/ coddle) to obtain a gene model and to identify the region that would have the highest likelihood to be functionally affected by EMS mutagenesis. The software PARSESNP (Project Aligned Related Sequences and Evaluate SNPs; http://www.proweb.org/parsesnp/, Taylor and Greene (2003) Nucleic Acids Res. 31: 3808-3811) was used to predict the putative severity of each identified mutation.

## Claims

1. Barley plant comprising a nucleotide substitution on nucleotide position 856 of the nucleic acid sequence coding for a Vrs protein, the nucleotide numbering referring to the nucleic acid sequence depicted in SEQ ID No. 1.

2. Barley plant according to claim 1, wherein the nucleotide substitution is a G to A substitution.

3. Barley plant according to claim 1 or 2, which comprises the nucleic acid sequence depicted in SEQ ID No. 2.

4. Barley plant according to any of the preceding claims, the seed of which being deposited with NCIMB under Accession number 41602.

5. Hybrids of the barley plant according to any of the preceding claims, the hybrids comprising a nucleotide substitution on nucleotide position 856 of the nucleic acid sequence coding for a Vrs protein, the nucleotide numbering referring to the nucleic acid sequence depicted in SEQ ID No. 1.

6. Parts of the barley plant according to any of claims 1 to 4, the parts comprising a nucleotide substitution on nucleotide position 856 of the nucleic acid sequence coding for a Vrs protein, the nucleotide numbering referring to the nucleic acid sequence depicted in SEQ ID No. 1.

7. Use of the barley plant according to any of claims 1 to 4, the hybrids of claim 5 or the parts of the plant according to claim 6 for the manufacture of a malt or a beverage.

8. Use according to claim 7, wherein the beverage is beer.

9. Barley plant seed deposited with NCIMB under Accession number 41602.

10. Method for obtaining six row-type barley plants, comprising the steps of:
a) mutagenizing barley seed; and
b) identifying barley seeds comprising a nucleotide substitution on nucleotide position 856 of the nucleic acid sequence coding for a Vrs protein, the nucleotide numbering referring to the nucleic acid sequence depicted in SEQ ID No. 1.

11. Method according to claim 10, wherein step c) comprises the steps of:
aa) amplifying a nucleic acid sequence comprising nucleotide 856 of the nucleic acid sequence coding for a Vrs protein, the nucleotide numbering referring to the nucleic acid sequence depicted in SEQ ID No. 1, from a nucleic acid sample obtained from a barley plant; and
bb) determining whether the nucleic acid sequence coding for a Vrs protein comprises a nucleotide substitution on position 856, the nucleotide numbering referring to the nucleic acid sequence depicted in SEQ ID No.1.

12. Method for identifying six row-type barley plants comprising identifying plants which comprise a nucleotide substitution on nucleotide position 856 of the nucleic acid sequence coding for a Vrs protein, the nucleotide numbering referring to the nucleic acid sequence depicted in SEQ ID No. 1.

13. Method according to claim 12, comprising the steps of:
aa) amplifying a nucleic acid sequence comprising nucleotide 856 of the nucleic acid sequence coding for a Vrs protein, the nucleotide numbering referring to the nucleic acid sequence depicted in SEQ ID No. 1, from a nucleic acid sample obtained from a barley plant; and
bb) determining whether the nucleic acid sequence coding for a Vrs protein comprises a nucleotide substitution on position 856, the nucleotide numbering referring to the nucleic acid sequence depicted in SEQ ID No.1.

14. A primer set for amplifying a nucleic acid sequence comprising nucleotide 856 of the nucleic acid sequence coding for a Vrs protein, the nucleotide numbering referring to the nucleic acid sequence depicted in SEQ ID No. 1, comprising a nucleic acid molecule comprising the nucleic acid sequence according to SEQ ID NO. 3 and a nucleic acid molecule comprising the nucleic acid sequence according to SEQ ID NO. 4.

15. Isolated nucleic acid molecule comprising a nucleic acid sequence selected from the group consisting of:
a) a nucleic acid sequence according to SEQ ID No. 2;
b) nucleic acid sequences hybridising to a complementary strand of the nucleic acid sequence according to SEQ ID No. 1 under stringent conditions and having a nucleotide substitution at position 856 of the nucleic acid sequence coding for a Vrs protein, the nucleotide numbering referring to the sequence depicted in SEQ ID No. 1; and
c) nucleic acid sequences which are at least 70 % homologous to the nucleic acid sequence shown in SEQ ID No. 1 and having a nucleotide substitution at position 856 of the nucleic acid sequence coding for a Vrs protein, the nucleotide numbering referring to the sequence depicted in SEQ ID No. 1.
